# EUROPEAN PATENT APPLICATION

(11) **EP 4 523 611 A1**
(43) Date of publication of application: **19.03.2025**
(21) Application number: 23382931.6
(22) Date of filing: 13.09.2023
(51) Int. Cl.: A61B 5/00, A61B 18/12, G01B 9/02017, G01N 21/23

(54) **METHODS, DEVICES, AND SYSTEMS FOR DETECTING MUSCLE FIBER ALIGNMENT**

(71) Applicant: Medlumics S.L., 28760 Tres Cantos, Madrid (ES)
(72) Inventor: Herranz, David, 28760 Tres Cantos - Madrid (ES); Duperron, Matthieu, 28760 Tres Cantos - Madrid (ES); Bailleul, Christophe, 28760 Tres Cantos - Madrid (ES)
(74) Representative: DTS Patent- und Rechtsanwälte PartmbB

(57) **Abstract**

Described herein are systems and methods for determining information about relative or absolute muscle fiber alignments in the depth of a tissue using Polarization-Sensitive Optical Coherence Reflectometry or Polarization-Sensitive Optical Coherence Tomography methods. The information about muscle fiber alignments of tissue is used to determine parameters for pulsed field-based ablation protocol.

## Description

### FIELD

Embodiments of the present disclosure relate to methods, devices, and systems for determining muscle fiber alignment in tissue using polarization-sensitive (PS) optical coherence interferometry (such as polarization-sensitive optical coherence tomography (PS-OCT) and polarization-sensitive optical coherent reflectometry (PS-OCR)) for performing lesion ablation.

### BACKGROUND

Ablation is a medical technique for producing tissue scars. It is used to help treat different pathologies including cancer, Barret's esophagus, or cardiac arrhythmias, among others. In some cases, various energy sources may be utilized for ablation, including pulsed electric fields, cryogenic cooling for cryoablation, radiofrequency, microwave, laser, ultrasound, and the like. For example, for an ablation procedure to treat Atrial Fibrillation, the electro-physiologist (EP) uses a catheter to deliver energy in specific locations of the heart to create transmural lines of ablation that will isolate electrically targeted areas of the heart walls. In particular, irreversible electroporation (IRE) or pulsed field ablation (PFA) has been gaining the interest of EPs and manufacturers, thanks to its reduced incidence of procedure-related adverse events, mainly thermal damages.

PFA uses electromagnetic pulses to generate non-thermal lesions during the ablation process. PFA may use electrodes to cause irreversible electroporation of the cells within it, ultimately leading to cell death. A high-intensity pulsed electric field for short periods of time (micro- or nanoseconds) results in electroporation, a process in which an applied electric field creates pores in cell membranes. Pore formation then leads to membrane permeabilization, which can be reversible or irreversible, depending on the parameters of the applied PFA. In reversible electroporation (RE), cells will remain viable, whereas in IRE, cells, and tissues are not viable due to activation of the programmed cell death cascade.

### BRIEF SUMMARY

In elongated muscle cells, it is interesting to know the orientation of the muscle fibers with respect to the electromagnetic field generated during PFA (also referred to as "coherent sine-burst electroporation (CSE)") delivering, since the threshold of irreversible electroporation in asymmetric systems is highly dependent on the orientation of this field. In the embodiments presented herein, methods and systems are described for determining a configuration of a fiber alignment of tissue by performing optical interferometry detection.

In an embodiment, a method of determining a configuration of a fiber alignment of the tissue is described. The method includes generating a beam of coherent light, coupling the beam of coherent light into an optical interferometer configured to deliver a first portion of the beam of coherent light to a tissue, and providing an output signal based on interference between reflected light from the tissue and a second portion of the beam of coherent light. The method further includes determining the polarization of the beam of coherent light and determining a birefringence of the tissue based on the output signal. The method further includes determining a configuration of a fiber alignment of the tissue based on the birefringence of the tissue and the polarization of the beam of coherent light.

In another embodiment, a method of determining a configuration of a fiber alignment of the tissue is described. The method includes generating a beam of coherent light, coupling the beam of coherent light into an optical interferometer configured to deliver a first portion of the beam of coherent light to a tissue, and providing an output signal based on interference between reflected light from the tissue and a second portion of the beam of coherent light. The method further includes adjusting the polarization of the beam of coherent light and determining a change of the output signal when adjusting the polarization of the beam of coherent light. The method further includes determining a configuration of a fiber alignment of the tissue according to the change of the output signal and the polarization of the beam of coherent light.

In another embodiment, a method of determining a configuration of a fiber alignment of the tissue is described. The method includes generating a beam of coherent light, coupling the beam of coherent light into an optical interferometer configured to deliver a first portion of the beam of coherent light via an optical port to a tissue, and providing an output signal based on an interference between a reflected light from the tissue and a second portion of the beam of coherent light. The method further includes determining a polarization of the beam of coherent light, adjusting the position of the optical port with respect to the tissue, and determining a change of the output signal when adjusting the position of the optical port. The method further includes determining a configuration of a fiber alignment of the tissue according to the change of the output signal and the polarization of the beam of coherent light.

Further features and advantages, as well as the structure and operation of various embodiments, are described in detail below with reference to the accompanying drawings. It is noted that the specific embodiments described herein are not intended to be limiting. Such embodiments are presented herein for illustrative purposes only. Additional embodiments will be apparent to persons skilled in the relevant art(s) based on the teachings contained herein.

### BRIEF DESCRIPTION OF THE DRAWINGS/FIGURES

The accompanying drawings, which are incorporated herein and form a part of the specification, illustrate embodiments of the present disclosure and, together with the description, further serve to explain the principles of the disclosure and to enable a person skilled in the pertinent art to make and use the disclosure.
FIG. 1 illustrates a diagram of an example system for ablation, according to embodiments of the present disclosure.
FIG. 2A illustrates an example diagram of a catheter, according to embodiments of the present disclosure.
FIG. 2B illustrates a diagram of an example distal section of a unipolar catheter, according to embodiments of the present disclosure.
FIG. 2C illustrates a diagram of an example distal section of a bipolar catheter, according to embodiments of the present disclosure.
FIGs. 3A and 3B illustrate cross sections of a catheter, according to embodiments of the present disclosure.
FIGs. 4A, 4B, and 4C illustrate examples of fiber alignments of the tissue with respect to a pulsed electric field, according to embodiments of the present disclosure.
FIG. 5A illustrates an image of an example sample of tissue having portions with different fiber alignments with respect to a pulsed electric field, according to embodiments of the present disclosure.
FIG. 5B illustrates, after applying the pulsed electric field, how different portions of the example sample of tissue change in time, according to embodiments of the present disclosure.
FIG. 6 illustrates a diagram of an example OCT and/or OCR system with multiple optical components, according to embodiments of the present disclosure.
FIG. 7A, 7B, and 7C illustrate examples of reflected optical signals of sample tissues with different fiber alignments with respect to a polarization of a probing signal, according to embodiments of the present disclosure.
FIG. 8A, 8B, and 8C illustrate diagrams of example schemes of implementing optical interferometry measurement to detect a fiber alignment of tissue, according to embodiments of the present disclosure.
FIG. 9 illustrates an example graphical user interface (GUI) showing predicted lesion depths probed by different optical components, according to embodiments of the present disclosure.
FIG. 10 illustrates an example method of conducting optical interferometry measurement to detect a fiber alignment of tissue, according to embodiments of the present disclosure.
FIG. 11 illustrates a block diagram of example components of the computer system, according to embodiments of the present disclosure.

Embodiments of the present disclosure will be described with reference to the accompanying drawings.

### DETAILED DESCRIPTION

Although specific configurations and arrangements are discussed, it should be understood that this is done for illustrative purposes only. A person skilled in the pertinent art will recognize that other configurations and arrangements can be used without departing from the spirit and scope of the present disclosure. It will be apparent to a person skilled in the pertinent art that this disclosure can also be employed in a variety of other applications.

It is noted that references in the specification to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases do not necessarily refer to the same embodiment. Further, when a particular feature, structure, or characteristic is described in connection with an embodiment, it would be within the knowledge of one skilled in the art to effect such feature, structure, or characteristic in connection with other embodiments whether or not explicitly described.

It should be noted that although this application may refer specifically to cardiac ablation, the embodiments described herein may target other pathologies as well. Further, embodiments may use additional energy sources for ablation, including but not limited to cryogenic, radiofrequency (RF), microwave, laser, ultrasound, and pulsed electric fields (PEFs). While the present disclosure primarily discusses embodiments in terms of catheters, a skilled artisan will recognize that similar techniques can be used with needles and the like.

Disclosed herein are embodiments of methods and systems with an ablation catheter for optical tissue evaluation and pulsed field ablation. In some embodiments, the ablation catheter can include a plurality of optical ports for evaluating target tissues. In some embodiments, the ablation catheter can include a plurality of electrodes for ablating target tissues. In some embodiments, the plurality of optical ports of the catheter may be configured to transmit beams of exposure radiation to a sample and receive one or more beams of scattered radiation that have been reflected or scattered from the sample. In some embodiments, the plurality of electrodes of the catheter may be configured to transmit PEF such that at least a portion of the sample is ablated. By utilizing the optical ports for transmission of the optical evaluation signals, the ablation catheter may provide a focused evaluation of the same target tissue that is being ablated in a single substrate that allows for both modalities.

Herein, the terms "electromagnetic radiation," "light," and "beam of radiation" are all used to describe the same electromagnetic signals propagating through the various described elements and systems.

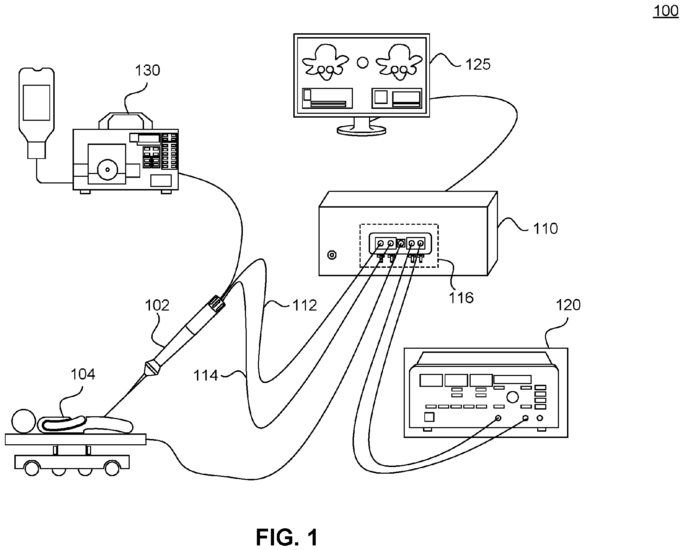

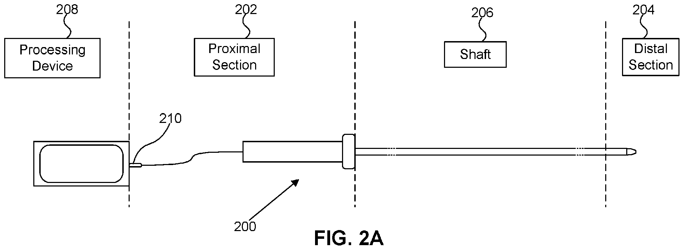

In some embodiments, an ablation catheter and console system described herein use OCT and/or OCR, or other methods to perform tissue ablations, track the formation of scar tissue in real-time, and monitor/verify lesion geometries and isolation by directly observing the scar pattern in tissue. FIG. 1 illustrates a diagram of an example system 100 for performing ablation according to embodiments of the present disclosure. The system 100 includes catheter 102, console 110, signal generator 120, display 125, and irrigation pump 130 (this pump will be optional as a function of the energy used). The catheter 102, console 110, signal generator 120, display 125, and irrigation pump 130 can be communicatively coupled together via wired and/or wireless connections. In some embodiments, catheter 102 can represent an exemplary embodiment of catheter 200 shown in FIG. 2A. In some embodiments, patient 104 is shown in FIG. 1 for illustrative purposes. It is understood that the embodiments described herein can be used in vivo and/or in vitro.

In some embodiments, catheter 102 can be positioned at a portion of tissue subject to ablation using energy generated by signal generator 120. In some embodiments, signal generator 120 can be an electronic device configured to generate radiofrequency (RF), cryogenic, laser or electroporation (e.g., pulsed electric field) signals for ablation. Signal generator 120 can be coupled to catheter 102 directly or via console 110, and can send energy to catheter 102 to ablate the portion of tissue at a selected tissue site. In some embodiments, the portion of tissue can be myocardial tissue, cardiac muscle tissue, skeletal tissue, or the like. Energy can be applied to the portion of tissue through optical viewports in the distal section of catheter 102. After applying the energy, structural changes in the tissue can be observed by acquiring optical signals via one or more optical viewports of catheter 102.

Console 110 can comprise a computing device configured to acquire the optical signals from catheter 102 and analyze the optical signals to detect changes in the optical properties of the tissue. In some embodiments, console 110 includes hardware (e.g., circuits), firmware, software, or any combination thereof to process the optical signals and perform further analysis. In some embodiments, console 110 can include an internal light source that provides a beam of coherent light. In some embodiments, console 110 can be coupled to an external light source that provides a beam of coherent light. In some embodiments, console 110 can send the beam of coherent light through an optical circuit within itself and the catheter 102 and into the tissue to monitor scar progression, contact condition between the tissue and catheter 102, and other characteristics of the tissue.

In some embodiments, console 110 can be referred to herein as a control console, processing device, and/or controller. Console 110 may be coupled to display 125, which can present results from the optical signal analysis and allow a user to select/view, modify, and/or control parameters related to the operation of catheter 102, console 110, signal generator 120, and/or irrigation pump 130.

In some embodiments, irrigation pump 130 may be coupled to catheter 102 via a tubing. In some embodiments, irrigation pump 130 may allow for fluid to be pumped through the tubing and released at the tissue site through catheter 102 (e.g., through optical viewports or through separate irrigation slits at the distal section of catheter 102). Fluid from the irrigation pump 130 may cool the distal section of catheter 102 and the surrounding tissue during ablation, and also flush away any debris during and/or after ablation.

In some embodiments, catheter 102 may be coupled to console 110 via one or more optical connections 112 and one or more electrical connections 114. Optical connections 112 may include single-mode optical fibers and/or multimode optical fibers that allow the acquisition and/or transmission of optical signals to and from catheter 102 and console 110 for further analysis. Electrical connection 114 may include wiring, pins, and/or components used for supplying power and energy from signal generator 120 to catheter 102 for ablation.

In some embodiments, the optical and electrical connections 112, and 114 may be connected to console 110 via a communication interface 116. Communication interface 116 may allow for the transmission of various signals (e.g., optical and electrical signals) between catheter 102 and console 110. In some embodiments, the communication interface 116 may include a connector that facilitates proper alignment of optical fibers between the catheter 102 and console 110.

FIG. 2A illustrates a catheter 200 according to embodiments of the present disclosure. Catheter 200 includes a proximal section 202, a distal section 204, and a shaft 206 coupled between proximal section 202 and distal section 204. In an embodiment, shaft 206 includes one or more radiopaque markers for navigation purposes. In one embodiment, catheter 200 includes a communication interface 210 between catheter 200 and a processing device 208. Communication interface 210 may include one or more one or more optical fibers and connectors between processing device 208 and catheter 200, as described herein. In other examples, communication interface 210 may include an interface component that allows wireless communication, such as Bluetooth, WiFi, cellular, and the like, to communicate with the catheter 200 or other processing components in a catheter system.

In an embodiment, shaft 206 and distal section 204 are disposable. As such, proximal section 202 may be reused by attaching a new shaft 206 and proximal section 204 each time a new procedure is to be performed. In another embodiment, proximal section 202 is also disposable.

Proximal section 202 may house various electrical and optical components used in the operation of catheter 200. In some embodiments, an optical source may be included within proximal section 202 to generate a source beam of radiation for optical evaluation. In some embodiment, the optical source can be an external optical source coupled to the proximal section via optical connections 112 in FIG. 1. The optical source may include one or more lasers, laser diodes, light emitting diodes (LEDs), or other kinds of optical coherent source. The beam of radiation generated by the optical source may have a wavelength within the infrared range. In one example, the beam of radiation has a central wavelength between about 1000 nm and 1600 nm. The optical source may be designed to output a beam of radiation at only a single wavelength, or it may be a swept source and be designed to output a range of different wavelengths. The generated beam of radiation may be guided towards distal section 204 via the optical transmission medium connected between proximal section 202 and distal section 204 within shaft 206. Some examples of optical transmission media include single-mode optical fibers and/or multimode optical fibers. In one embodiment, the electrical transmission medium and the optical transmission medium are provided by the same hybrid medium allowing for both electrical and optical signal propagation.

In an embodiment, proximal section 202 includes one or more components of an interferometer in order to perform coherence interferometry using the light generated from the optical sources. Due to the nature of interferometric data analysis, in an embodiment, the optical transmission medium used for guiding the light to and from distal section 204 does not affect the state and degree of light polarization. In another embodiment, the optical transmission medium can affect the polarization in a constant and reversible way. In some embodiments, catheter 200 may include an optical circuit with one or more elements configured to conduct optical spectroscopy. In such embodiments, at least part of the optical path may be made up of multi-mode optical transmission media (e.g. multi-mode optical fiber).

Proximal section 202 may include further interface elements with which a user of catheter 200 can control the operation of catheter 200. For example, proximal section 202 may include a deflection control mechanism that controls a deflection angle of distal section 204. The deflection control mechanism may include a mechanical movement of an element on proximal section 202, or the deflection control mechanism may use electrical and/or mechanical connections to control the movement of distal section 204. Proximal section 202 may include various buttons or switches that allow a user to control when the beams of radiation are transmitted from distal section 204, allowing for the acquisition of optical data. In some embodiments, proximal section 202 may include a deflection control mechanism for controlling one or more pull wires that are coupled to the distal section 204. In some embodiments, the deflection control mechanism and the one or more pull wires allow for steering of the distal section of catheter 200 in order to maneuver within and target specific tissue regions for ablation.

Distal section 204 includes a plurality of optical viewports. In some embodiments, the plurality of optical viewports may be referred to herein as orifices in the catheter tip. In an embodiment, one or more of the optical viewports are machined into the outer body of distal section 204. The optical viewports are distributed over the outside of distal section 104, resulting in a plurality of distinct viewing directions. In some embodiments, the optical viewports may transmit and collect light (e.g., optical signals) at various angles from the distal section 204. In an embodiment, each of the plurality of viewing directions is substantially non-coplanar. The optical viewports may also be designed with irrigation functionality to cool distal section 204 and surrounding tissue during ablation.

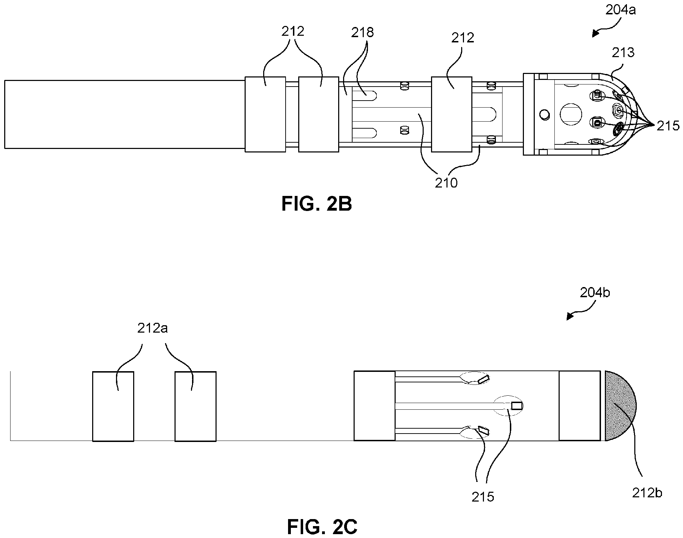

In some embodiments, a unipolar catheter can include a distal section having electrodes with one polarity. FIG. 2B illustrates a diagram of an example distal section of a unipolar catheter 204a, according to embodiments of the present disclosure. In some embodiments, the distal section of catheter 204a in FIG. 2B may represent an example embodiment of distal section 204 of catheter 200 shown in FIG. 2A. The distal section of catheter 204a includes a plurality of electrodes 212, ablation cap 213, a plurality of optical ports 215, one or more pull wire components 218, and irrigation tubing 210. In some embodiments, the distal section of catheter 204 may have a single electrode or multiple electrodes 212 having the same polarity to deliver ablation energy. In some embodiments, electrode 212 may be located at the very tip of distal section 204. In some embodiments, PFA energy may be used, and the electrode 212 configuration may be configured to deliver monophasic or biphasic pulse applications. In some embodiments, ablation cap 213 may also be an electrode and may be metallic. In some embodiments, ablation cap 213 may be referred to as a distal cap. In some embodiments, the plurality of optical ports 215 may be referred to herein as a plurality of optical viewports. In some embodiments, the pull wire components 218 may include an anchor and/or other components for allowing steering of the distal section of catheter 204 in order to maneuver within and target specific tissue regions for ablation. In some embodiments, irrigation tubing 210 may allow fluid to be guided along the catheter tip to cool tissue.

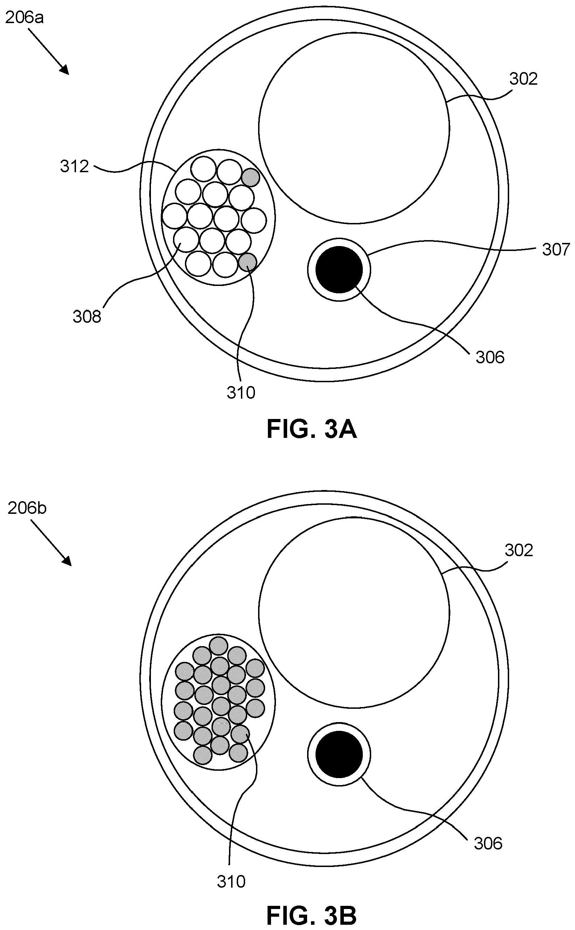

In some embodiments, the ablation cap 213 may include multiple optical ports 215, which may serve as optical windows or viewports for light beams from a plurality of optical fibers in the catheter. In some embodiments, optical ports 215 can be distributed in a nonplanar manner on a dome-shaped surface of ablation cap 213. In some embodiments, optical fibers (for example, optical transmission media 310 in FIGs. 3A and 3B) may be directed through the catheter shaft to lenses on the distal section of the catheter. In some embodiments, the optical fibers may be connected to the lenses by wafer-based wave-guide circuits that define the optical components at the catheter tip. In other embodiments, the optical fibers in the catheter tip may connect directly to the lenses, which focus the light into the tissue through the plurality of optical ports 215. In some embodiments, the lenses may be silicon or formed from another optically transparent material. In some embodiments, the lenses may also be coated to reduce reflections at interfaces or to allow optical index differences with surrounding tissue, blood, or fluid media.

In some embodiments, a bipolar catheter can include a distal section having electrodes with two polarities. FIG. 2C illustrates a diagram of an example distal section of a bipolar catheter 204b, according to embodiments of the present disclosure. In some embodiments, the distal section of catheter 204b in FIG. 2C may represent an example embodiment of distal section 204 of catheter 200 shown in FIG. 2A. The distal section of catheter 204b includes a plurality of optical ports 215, a plurality of electrodes 212a, and an electrode 212b. In some embodiments, electrode 212a can have a polarity opposite to that of electrode 212b. In some embodiments, electrodes 212a may be located at a middle region of distal section 204b. In some embodiments, electrode 212b may be located at the very tip of distal section 204b. In some embodiments, PFA energy may be used, and electrodes 212a and electrode 212b may be configured to deliver monophasic or biphasic pulse applications.

In some embodiments, the catheter tip may include a passive and fixed number of optics components (e.g., 15 fibers with 15 lenses attached), without any mechanical switching or scanning devices in the catheter itself. In some embodiments, movement or rotation of optical elements may allow for scanning in different directions in the tissue. In some embodiments, the plurality of optical ports or viewports in the catheter may have various orientations in the catheter tip, in which each output beam directed from each viewport in the catheter may face a different direction. For example, one output beam may be directed forward, seven output beams may be directed at 45° concerning tissue, and seven output beams may be directed at 90° concerning tissue. In some embodiments, there may be any number of beams, viewports, or orientations of the viewports in the catheter tip.

FIGs. 3A and 3B illustrate cross-section views of shaft 206, according to embodiments of the present disclosure. Shaft 206 may include all of the elements interconnecting proximal section 202 with distal section 204. Shaft 206a illustrates an embodiment that houses multiple channels/lumens, including an irrigation channel 302, a cabling channel 312, and a channel for deflection mechanisms 307. Through these channels, 307, 312, 302, deflection mechanism 306, electrical connections 308, and optical transmission medium 310, and cooling fluid may be at least partially housed or transported. In some configurations, a protective cover wrapped around both electrical connections 308 and optical transmission media 310 may be used. In other embodiments, optical transmission media 310 and components may be located within a protective cover that is separate from the protective cover in which the electrical connection 308 is housed. In some embodiments, electrical connections 308 may be used to provide signals to optical modulating components located in distal section 204. In some embodiments, electrical connections 308 may connect to electrodes 212 of FIG. 2B to deliver ablation energy. In some embodiments, one or more optical transmission media 310 guide light generated from the optical source (exposure light) towards distal section 204, while another subset of optical transmission media 310 guides light returning from distal section 204 (scattered or reflected light) back to proximal section 202. In another example, the same one or more optical transmission media 310 guides light in both directions. In some embodiments, the optical transmission medium 310 comprises one or more single-mode optical fibers and/or multimode optical fibers.

Irrigation channel 302 may be a hollow tube used to guide cooling fluid towards distal section 204. Irrigation channel 302 may include heating and/or cooling elements disposed along the channel to affect the temperature of the fluid. In another embodiment, irrigation channel 302 may also be used as an avenue for drawing fluid surrounding distal section 204 back towards proximal section 302.

Deflection mechanism 306 may include electrical and/or mechanical elements designed to provide a signal to distal section 204 to change the deflection angle of distal section 204. The deflection system enables guidance of distal section 204 by actuating a mechanical control placed in proximal section 202, according to an embodiment. This system may be based on a series of aligned and uniformly spaced cutouts in shaft 206 aimed at providing unidirectional deflection of distal section 204, in combination with a wire that connects the deflection mechanism control in proximal section 202 with the catheter tip at distal section 204. In this way, a certain movement of the proximal section may be projected to the distal section. Other embodiments involving the combination of several control wires attached to the catheter tip may enable the deflection of the catheter tip along different directions.

FIG. 3B illustrates a cross-section of shaft 206b. Shaft 206b depicts an embodiment having most of the same elements as shaft 206a from FIG. 3A, except that there are no electrical connections 308. Shaft 206b may be used in situations where modulation (e.g., multiplexing) of the generated beam of radiation is performed in proximal section 202 or distal section 204.

Referring back to FIGS. 2B and 2C, when performing an ablation treatment, ablation energy (e.g., PFA energy) may be delivered to electrodes 212 or 212a in distal section 204 of the catheter 200 and be applied to a target tissue to be treated. A pulsed electric field vector (PEF vector) of the PFA energy may be affected by various factors, such as the geometrical shapes of the electrodes and relative positions of the electrodes. In some embodiments, the effect of the ablation energy applied on the target tissue and surroundings depends on various factors, and parameters determining the amount of ablation energy need to be carefully chosen, such as the amplitude and the duration of the pulsed electric field, to effectively treat the target tissue while minimizing the damage to surrounding tissues. In particular, the fiber alignment of the target tissue concerning the PEF is an important factor to be considered for determining the parameters of the PFA energy when performing an ablation treatment.

Specifically, to reach the electroporation irreversibility barrier, the intensity of the applied electric field must be above a certain threshold. As the applied electric field is a vector, its orientation concerning the cell membrane is important in cardiac cells having an elongated shape. It is therefore useful to know the orientation of the muscle fibers concerning the applied field to achieve the optimized electroporation thresholds. Having the capability to directly assess the orientation of the muscle fibers concerning the applied field would enable clinicians to conduct safer, more reliable, and durable ablation treatment.

The information about the orientation of the muscle fibers concerning the applied field is not only useful at the endocardial surface with which the electrodes are placed in contact but also through the depth of the tissue. The implementation of ablation requires consistent and accurate detection of the tissue to be treated and the surrounding environment. In some cases, optical coherence interferometry, such as OCT or OCR, can be used for measuring various parameters of the tissue samples, such as the tomography and spectra of absorption, reflection, and birefringence. In some cases, these parameters are related to the orientation of the muscle fibers of the tissue, and vary during the process of the ablation treatment, requiring real-time detection and analysis to provide reliable information and guidance to the ablation treatment.

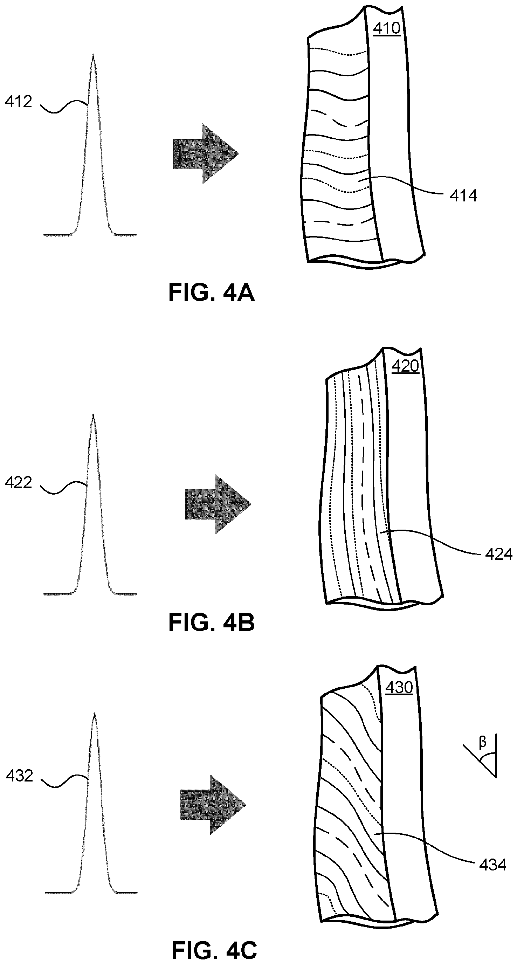

FIGs. 4A, 4B, and 4C illustrate examples of fiber alignments of the tissue concerning a PEF, according to embodiments of the present disclosure. In FIG. 4A, a tissue 410 has fibers aligned substantially perpendicular to a PEF vector 412, as shown on a surface 414 of tissue 410, on which the PEF is incident. In some embodiments, the fiber alignment within tissue 410 can be substantially uniform. In some embodiments, the fiber alignment in tissue 410 can vary slightly at different locations within the tissue. In some embodiments, due to the variation of the fiber alignment in tissue 410, the effect of ablation can vary at different locations within tissue 410, depending on the local condition of the fiber alignment within tissue 410 concerning the PEF vector 412.

In FIG. 4B, a tissue 420 has fibers aligned substantially parallel to a PEF vector 422, as shown on a surface 424 of tissue 420, on which the PEF is incident. In some embodiments, the fiber alignment within tissue 420 can be substantially uniform. In some embodiments, the fiber alignment in tissue 420 can vary slightly at different locations within the tissue. In some embodiments, due to the variation of the fiber alignment in tissue 420, the effect of ablation can vary at different locations within tissue 420, depending on the local condition of the fiber alignment within tissue 420 concerning the PEF vector 422.

In FIG. 4C, tissue 430 has fibers aligned along a general orientation concerning a PEF vector 432 of ablation energy, as shown on surface 434 of tissue 430, on which the PEF is incident. An angle β between the fiber alignment of tissue 430 and the PEF vector 432 can be between 0° and 90°. In some embodiments, the fiber alignment within tissue 430 can be substantially uniform. In some embodiments, the fiber alignment in tissue 430 can vary slightly at different locations within the tissue. In some embodiments, due to the variation of the fiber alignment in tissue 430, the effect of ablation can vary at different locations within tissue 430, depending on the local condition of the fiber alignment within tissue 430 concerning the PEF vector 432.

In the three different scenarios of FIGs. 4A, 4B, and 4C, the effect of ablation on tissues 410, 420, and 430 can be different and depends on various factors including the fiber alignment of the tissues concerning the pulsed electric fields. In some embodiments, the effectiveness of ablation on tissues 410 and 420 can differ by a substantial scale. For example, given the same ablation energy, the ablation performed on tissue 410 can be ineffective, while the ablation performed on tissue 420 can be effective, or vice versa. In some embodiments, depending on detailed characteristics of the type of tissue, a fiber alignment parallel to a PEF vector (e.g. the case of FIG. 4B) can cause more effective ablation than the fiber alignment perpendicular to the PEF vector (e.g. the case of FIG. 4A). In some other embodiments, depending on detailed characteristics of the type of tissue, a fiber alignment parallel to a PEF vector (e.g. the case of FIG. 4B) can cause less effective ablation than the fiber alignment perpendicular to the PEF vector (e. g. the case of FIG. 4A). In some embodiments, the effectiveness of ablation on tissue 430 is in a range between that on tissue 410 and tissue 420. In some embodiments, the information on the fiber alignment of a tissue can be used to determine parameters of the pulsed electric field to perform effective ablation while at the same time avoiding or minimizing thermal damages. In some embodiments, the information on the fiber alignment of the tissue can be used for fine-tuning proper values of the ablation parameters to perform effective ablation and minimize excessive energy delivery to a target tissue and/or surrounding tissues. In some embodiments, tracking the fiber alignment during the ablation procedure and how the fiber alignment changes during the ablation procedure, can guide the alignment of the catheter with respect to the fiber alignment of the tissue. In one example, as shown in FIG. 4A, if the perpendicular alignment between the fiber of tissue 410 and the PEF vector 412 causes less effective delivery of ablation energy to tissue 410, it may be helpful to tune ablation parameters, such as increasing the amplitude and/or a duration of the PEF, to perform effective ablation. In another example, as shown in FIG. 4B, if the parallel alignment between the fiber of tissue 420 and the PEF vector 422 provides more than enough ablation energy delivered to tissue 420, it may be helpful to tune ablation parameters, such as decreasing an amplitude and/or the duration of the PEF, to perform effective ablation while at the same time avoiding possible thermal damages to tissues surrounding tissue 420. In another example, as shown in FIG. 4C, if the general angle between the fiber alignment of tissue 430 and the PEF vector 432 causes less effective ablation, it may be helpful to adjust PEF vector 432, such as increasing or decreasing the angle β by adjusting the position/orientation of the electrodes on the catheter (for example, electrodes 212 of distal section 204 of catheter 200, as shown in FIGs. 2A and 2B), to improve the effectiveness of the ablation.

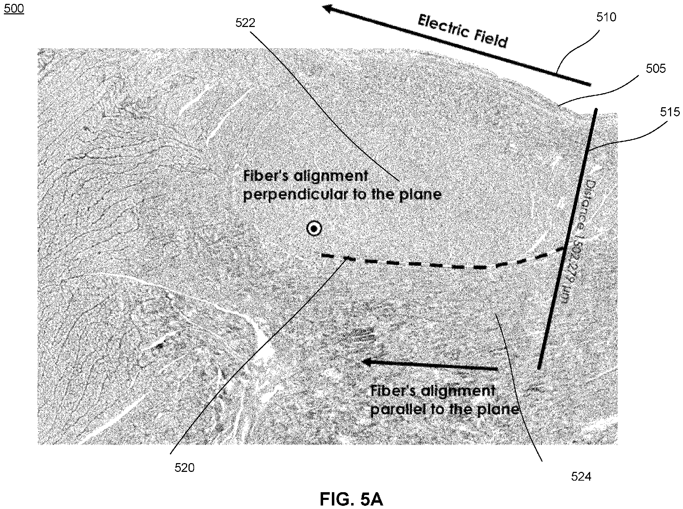

FIG. 5A illustrates an image of an example cross-section of a sample of a tissue having portions with different fiber alignments concerning a pulsed electric field, according to embodiments of the present disclosure. In FIG. 5A, a tissue 500 includes a first portion 522 and a second portion 524 separated by a boundary 520. Boundary 520 may be defined by changes in the alignment of fibers in tissue 500. An ablation operation is performed on tissue 500, using a pulsed electric field having a PEF vector 510 and penetrating tissue 500 from a surface 505 of tissue 500. FIG. 5A shows that the first portion 522 has a fiber alignment substantially perpendicular to PEF vector 510, whereas the second portion 524 has a fiber alignment substantially parallel to PEF vector 510. In some embodiments, due to their different fiber alignments concerning PEF vector 510, the first portion 522 and the second portion 524 can undergo different changing processes after the pulsed electric field is applied. For example, optical properties, such as birefringence of the first portion 522 and the second portion 524 can change differently (e.g., at different rates, or to a different stage of electroporation) after the pulsed electric field is applied. In some embodiments, the changing processes of the first portion 522 and the second portion 524 can be monitored to examine a correlation between fiber alignments in the first portion 522 and the second portion 524 concerning PEF vector 510.

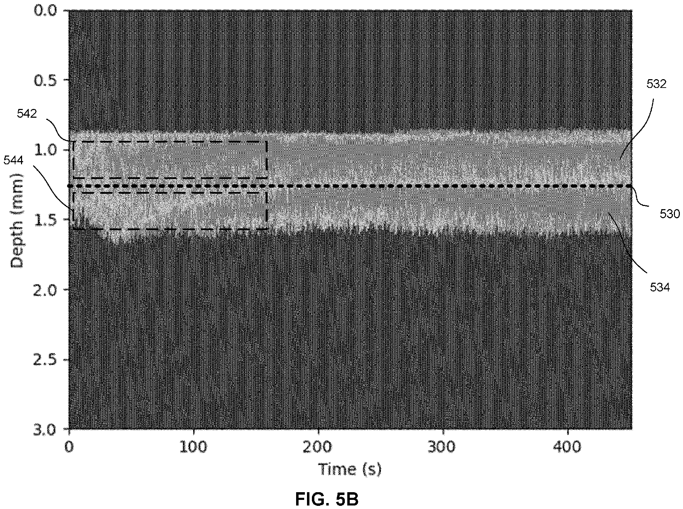

FIG. 5B illustrates, after applying the pulsed electric field, how the different portions of tissue 500 as shown in FIG. 5A change in time, according to embodiments of the present disclosure. In particular, FIG. 5B shows an evolution of birefringence of a cross-section 515 of the tissue 500 as shown in FIG. 5A. Here, after the pulsed electric field is applied, the birefringence evolves between 0 seconds beyond 400 seconds of time. A boundary 530 corresponds to the crossing point between boundary 520 and cross section cut 515 in FIG. 5A. Therefore, a first region 532 above boundary 530 corresponds to the birefringence of the first portion 522 as shown in FIG. 5A, and a second region 534 below boundary 530 corresponds to the birefringence of the second portion 524 as shown in FIG. 5A. Specifically, block 544 highlights the first 160 seconds of the second region 534, and shows the obvious change of the birefringence in the second portion 524, whereas another block 542, highlighting the first region 532 within the same period, shows insignificant change of the birefringence in the first portion 522. In some embodiments, the obvious change of the birefringence in the second portion 524, as highlighted by block 544, can indicate effective ablation in the second portion 524 due to the substantially parallel fiber alignment in the second region 524 concerning PEF vector 510. In some embodiments, the insignificant change of the birefringence in the first portion 522, as highlighted by block 542, can indicate ineffective ablation in the first portion 522 due to the substantially perpendicular fiber alignment in the first region 522 concerning PEF vector 510.

In some embodiments, knowing the information of the fiber alignment at different depths/locations of a tissue helps determine various ablation parameters to perform effective ablation at designated depths/locations of the tissue. In some embodiments, polarization-sensitive optical coherence tomography (PS-OCT) and/or polarization-sensitive optical coherence reflectometry (PS-OCR) can provide information of the fiber alignment at different depths/locations of the tissue.

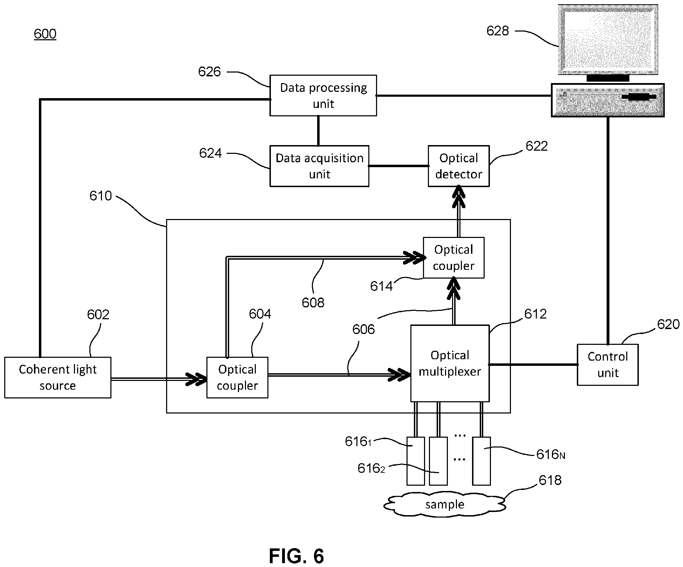

FIG. 6 illustrates a diagram of an example system 600 to perform OCT and/or OCR with multiple optical components, according to embodiments of the present disclosure. A coherent light source 602 provides a beam of coherent light. In some embodiments, coherent light source 602 can be an akinetic swept source or a VCSEL with tunable wavelength (for example, via a MEMS tunable filter). In some embodiments, coherent light source 602 can provide a beam of coherent light with a coherence length (as measured based on single path in air). For example, the beam of coherent light provided by the coherent light source 602 can have a coherence length of 0.1 mm to 1.0 mm, 1.0 mm to 1.0 cm, 1.0 cm to 10 cm, or greater than 10 cm. In some embodiments, coherent light source 602 can be a swept-source that generates a beam of coherent light having a wavelength periodically swept within a wavelength range around a central wavelength at a wavelength sweeping frequency. In some embodiments, the central wavelength can be infrared. In some embodiments, the central wavelength can be between 1000 nm and 1600 nm. For example, the central wavelength of the beam of coherent light can be about 1310 nm. In some embodiments, the wavelength range swept around the central wavelength can be between 30 nm and 70 nm. For example, the wavelength range swept around the central wavelength can be about 55 nm. In some embodiments, the wavelength sweeping frequency can be between 20 kHz and 100 kHz. For example, the wavelength sweeping frequency can be about 50 kHz. In some embodiments, coherent light source 602 can be an internal light source included in console 110 (as shown in FIG. 1) and coupled to optical transmission medium 310 (as shown in FIGs. 3A and 3B) of the catheter.

The beam of coherent light generated by coherent light source 602 is coupled to an interferometer 610. Interferometer 610 includes a first optical coupler 604 that couples a first portion of the beam of coherent light to a reference arm 608 and a second portion of the beam of coherent light to a sample arm 606. In some embodiments, sample arm 606 can be coupled to an optical multiplexer 612, which guides the second portion of the beam of coherent light into one or more optical components 616₁ - 616_{N}. Each of optical components 616₁ - 616_{N} includes an optical path that guides the second portion of the beam of coherent light to a location on sample tissue 618 to be inspected. Sample 618 can be a lesion, a tissue under an ablation process, or a test sample. Each of optical components 616₁ - 616_{N} can deliver the second portion of the beam of coherent light through one of the optical ports at the distal section of the catheter (for example, optical ports 215 in FIG. 2B). After illumination by the coherent light, a plurality of optical signals can return from sample 618, each corresponding to optical components 616₁ - 616_{N}. The plurality of optical signals depends on the condition of sample 618 and is correlated to the beam of coherent light. The plurality of optical signals are collected back by optical components 616₁ - 616_{N} and returned to sample arm 606 via optical multiplexer 612, and then combined with the first portion of the beam of coherent light in reference arm 608 by a second optical coupler 614. In some embodiments, a control unit 620 can be used to control optical multiplexer 612 to select one or more optical components 616₁ - 616_{N}, to which the second portion of the beam of coherent light is coupled. Control unit 620 can be connected to computer 628 to receive commands from and transmit data to computer 628.

In some embodiments, optical multiplexer 612 and optical components 616₁ - 616_{N} can be included inside catheter 102 of FIG. 1. In some embodiments, optical multiplexer 612 can be located inside console 110 of FIG. 1, and coupled to optical components 616₁ - 616_{N} in catheter 102 via optical connections 112. In some embodiments, the first optical coupler 604, the second optical coupler 614, and the reference arm 608 can be included in console 110.

Interferometer 610 provides a plurality of optical output signals by the second optical coupler 614, each corresponding to one of the optical components 616₁ - 616_{N}. Each of the optical output signals includes information about the interference between the reference arm and the sample arm coupled to one of the optical components 616₁ - 616_{N}. The optical output signals are detected by an optical detector 622. Optical detector 622 can convert the optical output signals into electrical signals containing information about the interference between the reference arm and the sample arm coupled to one of optical components 616₁ - 616_{N}, as functions of time.

In some embodiments, interferometer 610 can include optical fibers connecting optical coupler 604, optical coupler 614, optical multiplexer 612, and optical components 616₁ - 616_{N}. In some embodiments, interferometer 610 can be connected to coherent light source 602 and optical detector 622 via optical fibers. In some embodiments, the optical fibers can be single-mode fibers, multimode fibers, or a combination thereof. In some embodiments, some or all of the optical fibers can be polarization-maintaining (PM) fibers.

Since the wavelength of the beam of coherent light is being swept within a wavelength range under a sweeping frequency, the wavelength is also a function of time. Therefore, within each cycle of the wavelength sweeping, each of the electrical signals provided by optical detector 622 is also a function of the varying wavelength. A Fourier transform of the electrical signal from a wavelength domain (or equivalently, a wavenumber domain) into a real space domain (or equivalently, a depth domain) can provide signals related to optical properties of a location of sample 618 probed by an optical component, as functions of scan depth.

In some embodiments, optical detector 622 can process the optical output signals provided by interferometer 610 for effective measurement. For example, optical detector 622 can amplify the optical output signals, or filter the optical output signals to remove noise. To avoid signal distortion and provide high-quality electrical signals containing information about sample 618, optical detector 622 should have sufficient bandwidth to process and detect the optical output signals. In some embodiments, optical detector 622 can have a bandwidth of about 100 MHz.

In some embodiments, optical detector 622 can be located inside console 110 in FIG. 1, and the optical output signals can be transmitted to optical detector 622 via optical connections 112 and interface 116. In some embodiments, optical detector 622 can be located inside catheter 102 in FIG. 1, and the electrical signals provided by optical detector 622 can be transmitted to console 110 via electrical connections 114 and interface 116.

Electrical signals provided by optical detector 622 can further be sampled into digital data by a data acquisition unit 624, at a data sampling rate. In some embodiments, data acquisition unit 624 can be located inside console 110 in FIG. 1. To avoid signal distortion or loss of information in the signal, the data sampling rate of data acquisition unit 624 can be sufficiently higher than the bandwidth of the electrical signals provided by optical detector 622. In some embodiments, the data sampling rate can be greater than the bandwidth of the optical detector. For example, the data sampling rate can be greater than 100 MHz. In some embodiments, the data sampling rate can be between 0 and 175 MHz. In some embodiments, the data sampling rate can be between 0 and 400 MHz. In some embodiments, the data sampling rate of data acquisition unit 624 can be set by the k-clock of coherent light source 602 (for example, the k-clock of a VCSEL swept laser), such that the overall image range can automatically be ensured, and the sweeping nonlinearity can be compensated.

The data acquired by the data acquisition unit 624 is further transmitted to the data processing unit 626. In some embodiments, data processing unit 626 can be located in console 110 in FIG. 1. In some embodiments, data processing unit 626 can be a processing unit of computer 628. Data processing unit 626 can process the data and extract useful information from the data that can help assist in the analysis of the condition of sample 618. For example, data processing unit 626 can use a fast Fourier transform to convert the data as a function of wavelength into a plurality of signals as functions of scan depth from the surface of sample 618, with each of the plurality of signals corresponding to the condition of a location of sample 618 measured by one of the optical components 616₁ - 616_{N}. In some embodiments, data processing unit 626 can also be connected to coherent light source 602 to acquire the phase of the sweeping wavelength, so that the information of the wavelength is collected by data processing unit 626 for applying fast Fourier transform. In some embodiments, the plurality of signals can include information about refraction, birefringence, polarization, and/or other optical properties of sample 618. The plurality of signals as functions of scan depth can further be transmitted to display on a screen of computer 628.

In some embodiments, data processing unit 626 can also process the plurality of signals to extract information of interest to them. For example, data processing unit 626 can detect one or more reference features in the plurality of signals, and crop regions of interest in the plurality of signals, based on the location of one or more reference features.

In some embodiments, the coherent light delivered to sample 618 via optical components 616₁ - 616_{N} can be linearly polarized. In some embodiments, a polarization of the coherent light delivered to sample 618 can be known by either controlling the polarization of the coherent light or sampling the coherent light and measuring polarization. In some embodiments, information about the polarization of the coherent light can be known. In some embodiments, using PM fibers in system 600, the polarization of the coherent light sent to sample 618 can be fixed and is therefore known. In some embodiments, using single mode (SM) fibers in system 600, the polarization of the coherent light sent to sample 618 is not fixed, but can be computed by normalizing it using a known polarization state measured in system 600. In some embodiments, information about the relative polarization of coherent light can be known. In some embodiments, information about the polarization or the relative polarization of the coherent light helps extract the information of a fiber alignment of sample 618 by analyzing the output signals of optical detector 622 using data processing unit 626.

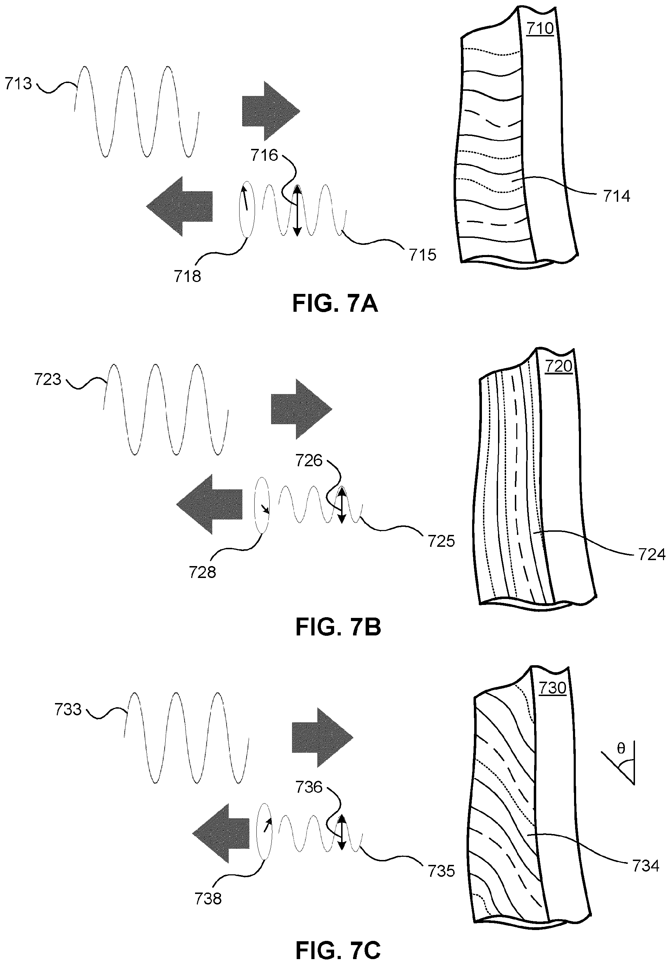

FIG. 7A, 7B, and 7C illustrate examples of reflected optical signals of tissues with different fiber alignments concerning a polarization of probing signals, according to embodiments of the present disclosure. In FIGs. 7A, 7B, and 7C, probing signals 713, 723, and 733 each can be a linearly polarized coherent light. In some embodiments, probing signals 713, 723, and 733 can be provided, respectively, to tissues 710, 720, and 730 by one of the optical components 616₁ - 616_{N} as shown in FIG. 6. In some embodiments, tissues 710, 720, and 730 can have different fiber alignments concerning polarizations of probing signals 713, 723, and 733. In some embodiments, after being probed by probing signals 713, 723, and 733, tissues 710, 720, and 730 can provide reflected signals 715, 725, and 735, respectively. In some embodiments, signals 715, 725, and 735 can be collected by optical components 616₁ - 616_{N} and transmitted back to interferometer 610 as shown in FIG. 6.

In FIG. 7A, tissue 710 can have a fiber alignment substantially perpendicular to the polarization of probing signal 713, as indicated by fiber alignments on an incident surface 714 of tissue 710. In FIG. 7B, tissue 720 can have a fiber alignment substantially parallel to the polarization of probing signal 723, as indicated by fiber alignments on an incident surface 724 of tissue 720. In FIG. 7C, tissue 730 can have a fiber alignment having a general angle θ concerning the polarization of probing signal 713, with 0° ≤ θ ≤ 90°, as indicated by fiber alignments on an incident surface 734 of tissue 730. In some embodiments, probing signals 713, 723, and 733 can have the same parameters, such as the same polarization and intensity. In some embodiments, due to the different fiber alignments concerning the polarizations of probing signals 713, 723, and 733, reflected signals 715, 725, and 735 can have different parameters, such as different polarizations and intensities. In some embodiments, different fiber alignments with respect to the polarizations of probing signals can cause different strengths of optical rotation, such that a polarization 718 of reflected signal 715, a polarization 728 of reflected signal 725, and a polarization 738 of reflected signal 735 can be different. In some embodiments, different fiber alignments with respect to the polarizations of probing signals can cause different diattenuation, such that an intensity 716 of reflected signal 715, an intensity 726 of reflected signal 725, and an intensity 736 of reflected signal 735 can be different. In some embodiments, different fiber alignments concerning the polarizations of probing signals can cause different diattenuation, such that a phase of reflected signal 715 concerning probing signals 713, a phase 727 of reflected signal 725 concerning probing signals 713, and a phase 737 of reflected signal 735 concerning probing signals 713 can be different. In some embodiments, the different reflected signals can cause different output signals provided by optical detector 622 as shown in FIG. 6, and can further be analyzed to extract information about the fiber alignment of the tissues. In some embodiments, with a certain fiber alignment, a reflected signal by a tissue is correlated with a polarization of a probing signal, and by varying the polarization of the probing signal and analyzing an output signal of optical detector 622 as a function of the polarization of the probing signal, comprehensive information about the tissue, such as the fiber alignment at different locations within the tissue, can be determined. In some embodiments, the correlation between the reflected signal by the tissue and the polarization of a probing signal can depend on the type of tissue. In some embodiments, data about the correlation between the reflected signal and the polarization of a probing signal can be analyzed using artificial intelligence technologies, such as machine learning and deep learning. In some embodiments, these data for different types of tissues can be collected to build a comprehensive database, which can provide effective and efficient schemes to acquire the information on fiber alignment of the tissues, and can further provide proper parameters for performing ablation.

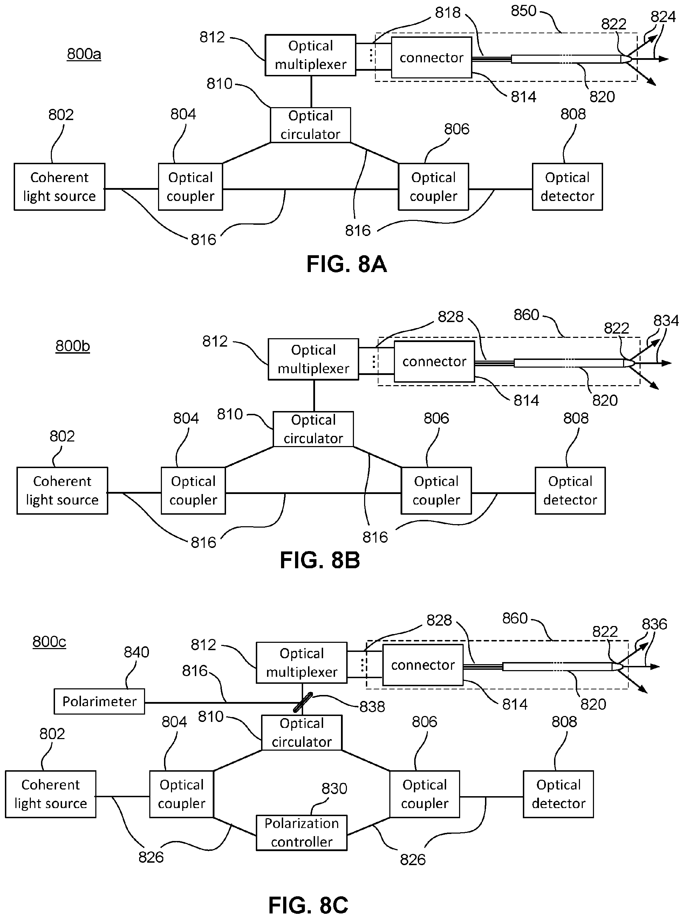

FIG. 8A, 8B, and 8C illustrate diagrams of exemplary schemes of implementing optical interferometry measurement to detect a fiber alignment of tissue, according to embodiments of the present disclosure. In some embodiments, the exemplary schemes can be implemented by polarization-sensitive OCR (PS-OCR) systems or polarization-sensitive OCT (PS-OCT) systems.

In FIG. 8A, a PS-OCR system 800a can include a coherent light source 802, a first optical coupler 804, a second optical coupler 806, an optical circulator 810, an optical multiplexer 812, and an optical detector 808, all of which are coupled by optical fibers 816. In some embodiments, optical fibers 816 can be polarization-maintaining (PM) optical fibers. PS-OCR system 800a can include a catheter 850. Catheter 850 can include a connector 814, a shaft 820, and a distal section 822. Coherent light source 802 can provide a polarized coherent light and transmit the polarized coherent light to the first optical coupler 804. The first optical coupler 804 can split the polarized coherent light into first and second portions of the polarized coherent light, transmit the first portion of the polarized coherent light to optical circulator 810 (e.g., the sample arm), and transmit the second portion of the polarized coherent light to the second optical coupler 806 (e.g., via the reference arm). Optical circulator 810 can transmit the first portion of the polarized coherent light to optical multiplexer 812. Optical multiplexer 812 can be connected to connector 814 of catheter 850 via the plurality of optical paths 818, and transmit the first portion of the polarized coherent light to catheter 850 by choosing one or more of the plurality of optical paths 818. In some embodiments, each of the plurality of optical paths 818 can include a PM optical fiber. Inside catheter 850, the first portion of the polarized coherent light can be passed through shaft 820 and distal section 822 and can be delivered to a sample via one or more optical ports on distal section 822 in one or more directions, as indicated by lights 824. A reflected light returned from the sample can be captured by catheter 850 and delivered back to optical circulator 810 via one or more of the plurality of optical paths 818 and optical multiplexer 812. Optical circulator 810 can transmit the reflected light to the second optical coupler 806, where the reflected light interferes with the second portion of the polarized coherent light, and generates an interference signal detected by optical detector 808.

In some embodiments, PS-OCR system 800a can include the same or similar elements in FIG. 6, For example, coherent light source 802 can be the same as or similar to coherent light source 602, the first optical coupler 804 can be the same as or similar to optical coupler 604, the second optical coupler 806 can be the same as or similar to optical coupler 614, optical multiplexer 812 can be the same as or similar to optical multiplexer 612, and optical detector 808 can be the same as or similar to optical multiplexer 622. In some embodiments, catheter 850 can be the same as or similar to catheter 200 in FIG. 2A. In some embodiments, the first portion of the polarized coherent light delivered to the sample can be the same as or similar to probing signal 713 in FIG. 7A, probing signal 723 in FIG. 7B, or probing signal 733 in FIG. 7C.

Referring to Fig. 8A, since optical fibers 816 can be PM optical fibers, and each of the plurality of optical paths 818 can include a PM optical fiber, the polarized coherent light provided by the coherent light source 802 can have a polarization state maintained when delivered through PS-OCR system 800a so that the first portion of the polarized coherent light and the lights 824 delivered to the tissue can have the same polarization state as the polarized coherent light. In some embodiments, the polarization state of lights 824 can be controlled and adjusted when probing a muscle fiber alignment in the tissue. For example, in some embodiments, the polarization of the polarized coherent light can be rotated, by rotating an output of the coherent light source around a propagation axis of the polarized coherent light. In some embodiments, the polarization of the polarized coherent light can be controlled by using additional optical elements (e.g., polarizers). In some embodiments, the polarization state of light 824 delivered to the tissue can be controlled by adjusting the position of optical ports on the distal section 822. In some embodiments, the polarization state of light 824 delivered to the tissue can be recorded and used for analyzing the muscle fiber alignment of the tissue. In some embodiments, by changing the polarization state (e.g., scanning the polarization state in a range between 0° and 90°), the interference signal as a function of the polarization state can be obtained and used to calculate the muscle fiber alignment of the tissue. In some embodiments, analyzing the interference signal as a function of the polarization state can be done by a data processing unit (e.g., the data processing unit 626 in FIG. 6). In some embodiments, algorithms, such as fast Fourier transform and Stokes vector analysis, can be applied to process the interference signal as a function of the polarization state, and calculate the muscle fiber alignment of the tissue at different locations (e. g., different depths) within the tissue. In some embodiments, a relative change in the muscle fiber alignment at different depths can be calculated. In some embodiment, the interference signal as a function of the polarization state can be processed to construct a three-dimensional (3D) configuration of the fiber alignment of the tissue.

FIG. 8B illustrates another scheme using a PS-OCR system 800b different from PS-OCR system 800a in FIG. 8A. PS-OCR system 800b can include some elements the same as they are in PS-OCR system 800a, such as coherent light source 802, the first optical coupler 804, the second optical coupler 806, optical circulator 810, optical multiplexer 812, and optical detector 808. These elements in PS-OCR system 800b can be coupled by optical fibers 816. In some embodiments, optical fibers 816 can be polarization-maintaining (PM) optical fibers.

Similar to PS-OCR system 800a, PS-OCR system 800b can include a catheter 860. Catheter 860 can include some elements the same as they are in catheter 850, such as connector 814, shaft 820, and distal part 822. Optical multiplexer 812 can be connected to connector 814 of catheter 860 via the plurality of optical paths 828 and transmits the first portion of the polarized coherent light to catheter 860 by choosing one or more of the plurality of optical paths 828. In some embodiments, different from the plurality of optical paths 818 in FIG. 8A, each of the plurality of optical paths 828 can include a single mode (SM) optical fiber. In some embodiments, using SM optical fibers instead of PM optical fiber can reduce the cost of manufacturing catheter 860. In PS-OCR system 800b, the polarization state is maintained when delivered through optical fibers 816 but is not maintained in the plurality of optical paths 828, such that a polarization state of lights 834 delivered to the tissue is not the same as the polarization state of the polarized coherent light from source 802. In some embodiments, the SM optical fiber can introduce optical rotation to the polarization state of the first portion of polarized coherent light, so that there is a relative difference between the polarization state of the first portion of polarized coherent light before entering the plurality of optical paths 828 and the polarization state of lights 834 delivered at the tissue. The polarization state of lights 834 can also be referred to as a relative polarization state. In some embodiments, a relative change in the muscle fiber alignment at different depths can be calculated. In some embodiments, the relative polarization state at the output of catheter 860 can be measured to compute and numerically compensate for the polarization change introduced by the SM optical fiber.

In some embodiments, the relative polarization state of light 834 can be recorded and used for analyzing the muscle fiber alignment of the tissue. In some embodiments, the relative polarization state of lights 834 together with the interference signal detected by optical detector 808 can be analyzed together to calculate the muscle fiber alignment of the tissue. In some embodiments, the interference signal as a function of the relative polarization state of lights 834 can be analyzed to calculate the muscle fiber alignment of the tissue. In some embodiments, analyzing the interference signal as a function of the relative polarization state can be performed by a data processing unit (e.g., data processing unit 626 in FIG. 6). In some embodiments, algorithms, such as fast Fourier transform, can be applied to process the interference signal as a function of the relative polarization state of lights 834, and calculate the muscle fiber alignment of the tissue at different locations (e. g., different depths) within the tissue. In some embodiments, a relative change in the muscle fiber alignment at different depths can be calculated. In some embodiments, the interference signal as a function of the relative polarization state of lights 836 can be processed to construct a three-dimensional (3D) configuration of the fiber alignment of the tissue.

FIG. 8C illustrates another scheme using a PS-OCR system 800c different from PS-OCR system 800a in FIG. 8A and PS-OCR system 800b in FIG. 8B. PS-OCR system 800c can include some elements the same as they are in PS-OCR system 800a and PS-OCR system 800b, such as coherent light source 802, the first optical coupler 804, the second optical coupler 806, optical circulator 810, optical multiplexer 812, and optical detector 808. These elements of PS-OCR system 800c can be coupled by optical fibers 826. In some embodiments, PS-OCR system 800c can further include a polarization controller 830 coupled between the first optical coupler 804 and the second optical coupler 806 by optical fibers 826. In some embodiments, different from optical fibers 816 in PS-OCR system 800a and PS-OCR system 800b, optical fibers 826 in PS-OCR system 800c can be SM optical fibers. In some embodiments, using SM optical fibers instead of PM optical fibers can reduce the cost of manufacturing PS-OCR system 800c.

Similar to PS-OCR system 800b, PS-OCR system 800c can include catheter 860. Optical multiplexer 812 can be connected to connector 814 of catheter 860 via the plurality of optical paths 828 and transmits the first portion of the polarized coherent light to catheter 860 by choosing one or more of the plurality of optical paths 828. In some embodiments, similar to PS-OCR system 800b, each of the plurality of optical paths 828 can include a single mode (SM) optical fiber. In some embodiments, using SM optical fibers instead of PM optical fiber can reduce the cost of manufacturing catheter 860. In PS-OCR system 800c, the polarization state is not maintained when delivered through optical fibers 826 and in the plurality of optical paths 828, such that the polarization state of lights 836 delivered to the tissue is not the same as the polarization state of the polarized coherent light from source 802. In some embodiments, the SM optical fibers can introduce optical rotation to the polarization state of the polarized coherent light, so that there is a relative difference between the polarization state of the polarized coherent light provided by the coherent light source 802 and the polarization state of lights 836 delivered at the tissue. The polarization state of lights 836 can also be referred to as a relative polarization state.

In some embodiments, the relative polarization state of lights 836 can be compensated by a polarization reference signal. For example, in some embodiments, PS-OCR system 800c can further include a polarimeter 840 to provide the polarization reference signal as compensation to lights 836, so that optical rotations introduced by SM optical fibers can be compensated. In some embodiments, via an optical fiber 816, which is a PM optical fiber and maintains the polarization state of the polarization reference signal provided by polarimeter 840, polarimeter 840 can be coupled to an optical switch 838 placed along an optical path between optical multiplexer 812 and optical circulator 810, so that the compensation of polarization change can be introduced.

In some embodiments, the relative polarization state of lights 836 can be recorded and used for analyzing the muscle fiber alignment of the tissue. In some embodiments, the relative polarization state of lights 836 together with the interference signal detected by optical detector 808 can be analyzed together to calculate the muscle fiber alignment of the tissue. In some embodiments, the interference signal as a function of the relative polarization state can be analyzed to calculate the muscle fiber alignment of the tissue. In some embodiments, analyzing the interference signal as a function of the relative polarization state of lights 836 can be performed by a data processing unit (e.g., the data processing unit 626 in FIG. 6). In some embodiments, algorithms, such as fast Fourier transform and Stokes vector analysis, can be applied to process the interference signal as a function of the relative polarization state of lights 836 and calculate the muscle fiber alignment of the tissue at different locations (e. g., different depths) within the tissue. In some embodiments, a relative change in the muscle fiber alignment at different depths can be calculated. In some embodiment, the interference signal as a function of the relative polarization state of lights 836 can be processed to construct a three-dimensional (3D) configuration of the fiber alignment of the tissue.

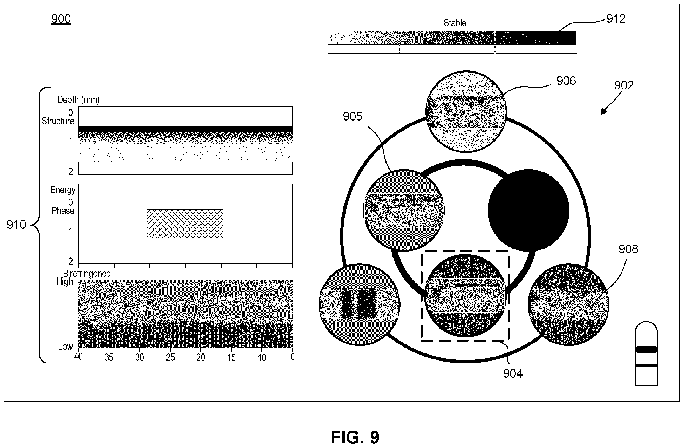

FIG. 9 illustrates an example graphical user interface (GUI) 900 showing predicted lesion depths, according to embodiments of the present disclosure. The GUI 900 provides optical measurement data, for example as processed by console 110, in real-time or near real-time for an ablation process. For example, GUI 900 may be presented on display 125 coupled to console 110 in FIG. 1. In another example, GUI 900 may be presented on the display of computer 628 in FIG. 6, according to the output of data processing unit 626. In some embodiments, the GUI 900 includes a front view 902 of the catheter tip showing different sections 904-906 corresponding to the various optical viewports in the catheter tip, each optical viewport corresponds to one of the optical components (for example, optical components 616₁-616_{N} in FIG. 6).

In some embodiments, the front view 902 of GUI 900 may show which optical viewports of the catheter tip are in contact with tissue and which beams from the different optical viewports are in operation. For example, the dark gray section 904 of the front view 902 may indicate a strong contact between the catheter and tissue, the light gray section 905 may indicate a minimal or intermediate contact between the catheter and tissue, and the white section 906 may indicate no contact. In some embodiments, the stability conditions of the contacts can be referred to as a continuous scale 912. In some embodiments, the different sections 904-906 may also indicate which beams are switched on or off for obtaining optical measurements from the tissue. In some embodiments, the dark gray sections 904 and light gray section 905 may indicate that the beams from the corresponding optical viewports are turned on, whereas the white sections 906 may indicate that the corresponding optical viewports are turned off.

In some embodiments, the GUI 900 may further include a plurality of tiles 908 each corresponding to the different sections 904-906 in the front view 902. Each tile 908 may represent the image resulting from processing, by the console, the optical signal, and/or optical measurements obtained from a respective optical viewport section in the catheter. In some embodiments, individual tiles 908 may be switched on or off (or may appear or disappear) based on a particular optical viewport section being active at a given time. In some embodiments, individual tiles 908 may provide information about the fiber alignment of a tissue probed by a corresponding optical component. In some embodiments, individual tiles 908 may present a configuration of the fiber alignment of the tissue at different depths. In some embodiment, individual tiles 908 may display a birefringence of tissue measured by a respective optical component. In some embodiments, individual tiles 908 may provide a 3D configuration of the fiber alignment of the tissue. In some embodiments, the GUI 900 may provide recommended parameters for ablation, based on analyzing the fiber alignment of the tissue. In some embodiments, the GUI 900 may include one or more graphs 910 showing ablation energy data (e.g., RF power), birefringence data, phase data, and predicted lesion depth data. In some embodiments, the GUI 900 may include one or more panels or indicators 912 that show the occurrence of a stable contact between the catheter tip and tissue, loss in birefringence, status of the ablation energy (e.g., on/off), and predicted lesion depths. In some embodiments, the GUI 900 may include one or more buttons or text boxes that allow user selection and/or customization of parameters selected for ablation or for operating the catheter during ablation.

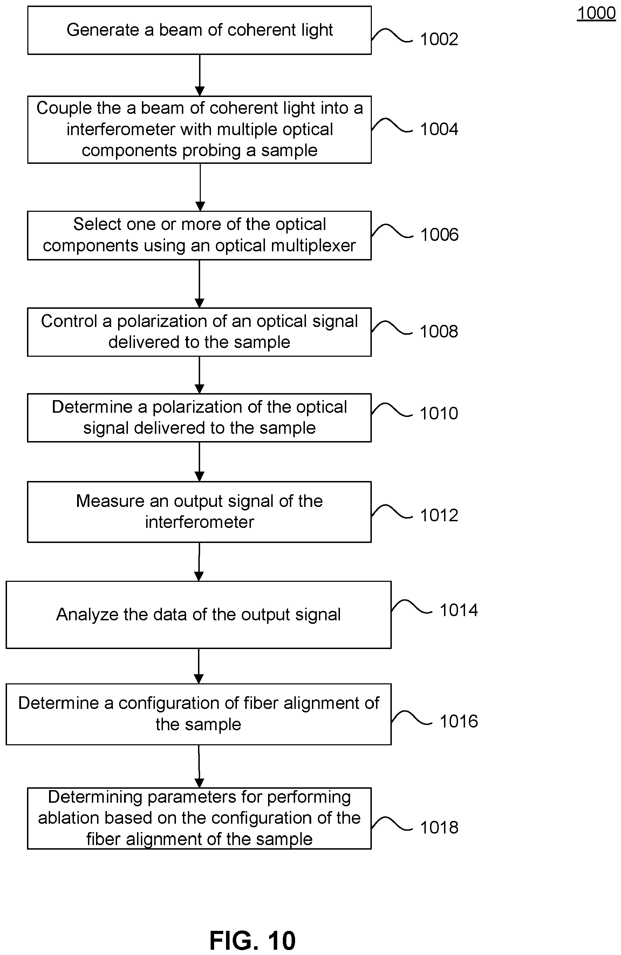

FIG. 10 illustrates an example method 1000 of performing optical interferometry measurement to detect a fiber alignment of tissue, according to embodiments of the present disclosure. In some embodiments, optical interferometry measurement can be performed by using a PS-OCR system, such as PS-OCR system 800a in FIG. 8A, PS-OCR system 800b in FIG. 8B, or PS-OCR system 800c in FIG. 8C. In some embodiments, the PS-OCR system can include an interferometer, such as interferometer 610 in FIG. 6. In some embodiments, the PS-OCR system can include a catheter probing a sample, for example, a lesion or a tissue under an ablation process), such as catheter 200 in FIG. 2A.

In step 1002, a beam of coherent light can be generated by a coherent light source (for example, coherent light source 602 in FIG. 6 or coherent light source 802 in FIGs. 8A-8C). In some embodiments, the beam of coherent light can be linearly polarized. In some embodiments, the beam of coherent light can have a wavelength periodically swept within a wavelength range around a central wavelength at a wavelength sweeping frequency. In some embodiments, the coherent light source can be a tunable vertical-cavity surface-emitting laser (VCSEL) or an akinetic swept source. In some embodiments, the central wavelength of the beam of coherent light can be between 1000 nm and 1600 nm. For example, the central wavelength of the beam of coherent light can be about 1310 nm. In some embodiments, the wavelength range swept around the central wavelength can be between 30 nm and 70 nm. For example, the wavelength range swept around the central wavelength can be about 55 nm. In some embodiments, the wavelength sweeping frequency can be between 20 kHz and 100 kHz. For example, the wavelength sweeping frequency can be about 50 kHz.

In step 1004, the beam of coherent light can be coupled into an optical interferometer (for example, interferometer 610 in FIG. 6) including a reference arm and a sample arm. In some embodiments, the sample arm can include a plurality of optical paths realized by a plurality of optical components (for example, optical components 616₁-616_{N} in FIG. 6) inspecting the sample. In some embodiments, the plurality of optical paths can include PM optical fibers or SM optical fibers. In some embodiments, the sample arm can include an optical multiplexer (for example, optical multiplexer 612 in FIG. 6) for selecting one or more optical paths onto which an optical signal is delivered to the sample via a catheter. In some embodiments, the optical signal can be a portion of the beam of coherent light,

In step 1006, one or more optical paths can be selected using the optical multiplexer. In some embodiments, the optical multiplexer can be controlled by a control unit (for example, control unit 620 in FIG. 6) to select one or more optical paths. In some embodiments, a chosen optical path can deliver an optical signal to the sample. In some embodiments, the optical signal delivered to the sample can be a portion of the beam of coherent light.

In step 1008, a polarization state of the optical signal delivered to the sample can be controlled. For example, in some embodiments, the polarization state of the beam of coherent light can be rotated, by rotating an output of the coherent light source along a propagation axis of the polarized coherent light. In some embodiments, the polarization state of the polarized coherent light can be controlled by using additional optical elements (e.g., polarizers or PM fibers). In some embodiments, a compensation of an optical rotation of the polarization state of the beam of coherent light can be introduced by a polarimeter, such as polarimeter 840 in FIG. 8C. In some embodiments, adjusting the positions of optical ports on the distal section of the catheter concerning the tissue can control the polarization state of the optical signal delivered to the sample.

In step 1010, a polarization of the optical signal delivered to the sample can be determined. In some embodiments, if the PS-OCR system maintains the polarization state of the beam of coherent light (e.g., optical fibers in the PS-OCR system are PM optical fibers, as shown in FIG. 8A), the polarization state of the optical signal can be determined by the polarization state of the beam of coherent light. In some embodiment, if the PS-OCR system does not maintain the polarization state of the beam of coherent light (e.g., some or all optical fibers in the PS-OCR system are SM optical fibers, as shown in FIG. 8B and 8C), the polarization state of the optical signal can be determined by measuring a relative polarization state.

In step 1012, an output signal provided by the interferometer is measured by an optical detector, such as optical detector 808 in FIGs. 8A-8C. The output signal is due to an interference of a reflected signal returned from the sample and a portion of the beam of coherent light in a reference arm of the interferometer. In some embodiments, the output signal can be measured as a function of different parameters. In one example, the output signal can be measured under different polarization states of the beam of coherent light. In another example, the output signal can be measured while adjusting the positions of optical ports on the distal section of the catheter concerning the tissue. In another example, a change of the output signal can be measured while adjusting the positions of optical ports on the distal section of the catheter concerning the tissue. In some embodiments, the optical detector converts the output signal into an electrical signal, and data about the electrical signal can be obtained by sampling the electrical signal under a data sampling rate. In some embodiments, a data acquisition unit (for example, data acquisition unit 624 in FIG. 6) can be used to acquire the data.

In step 1014, the data can be processed and analyzed by a data processing unit (for example, data processing unit 626 in FIG. 6). The data processing unit can compute a plurality of signals from the data, and each of the plurality of signals corresponds to one of the plurality of optical paths. In some embodiments, the data processing unit can compute the plurality of signals using a fast Fourier transform, so that the output signal as a function of the swept wavelength is converted into a signal about optical properties of the sample as functions of a depth inside the sample. In some embodiments, the optical properties of the sample include a birefringence of the sample. In some embodiments, information of interest in the plurality of signals can be processed by the data processing unit. In some embodiments, the data processing unit can crop regions of interest in the signal. In some embodiments, the data processing unit can implement a method of peak detection to detect one or more reference features and the regions of interest.

In step 1016, the signal about the optical properties of the sample, together with the information about the polarization of the optical signal delivered to the sample, can be used to determine a configuration of fiber alignment of the sample. In some embodiments, the configuration of fiber alignment of the sample can be calculated by the data processing unit. In some embodiments, the configuration of fiber alignment of the sample can be calculated by a computer, such as computer 628 in FIG. 6. In some embodiments, the configuration of fiber alignment of the sample as a function of depth inside the sample can be determined. In some embodiments, a three-dimensional (3D) configuration of the fiber alignment of the sample can be calculated. In some embodiments, the configuration of fiber alignment of the sample is a relative configuration between different parts of the sample tissue. In some embodiments, artificial intelligence technologies, such as machine learning and deep learning, can be used to determine the absolute configuration of the fiber alignment of the sample. In some embodiments, the configuration of the fiber alignment of the sample can be displayed as a plurality of tomographic images on a screen (for example, screen 125 in FIG. 1 or the screen of computer 628 in FIG. 6) for analysis and/or diagnosis of the sample. Regions of interest corresponding to different optical components can be displayed on different sections (for example, sections 904-906 in FIG. 9) on the screen on a real time basis.

In step 1018, based on the relative or absolute configuration of fiber alignment of the sample, parameters for performing ablation on the sample can be determined. In some embodiments, a power, a duration, and/or a PEF vector to be used for the PFA-based ablation protocol can be determined or fine-tuned. In some embodiments, the locations of electrodes on a catheter used for performing the ablation can be adjusted. In some embodiments, relative positions between the electrodes can be adjusted. In some embodiments, artificial intelligence technologies, such as machine learning and deep learning, can be used to optimize the parameters for the PFA-based ablation protocol. In some embodiments, the optimized parameters can provide an effective ablation operation on the sample while minimizing thermal damages or other adverse events on the sample and/or the regions surrounding the sample.

In some embodiments, method 1000 is performed during an ablation procedure to monitor in real time a change in the configuration of fiber alignments of a sample, and actively adjust the parameters for the PFA-based ablation procedure.

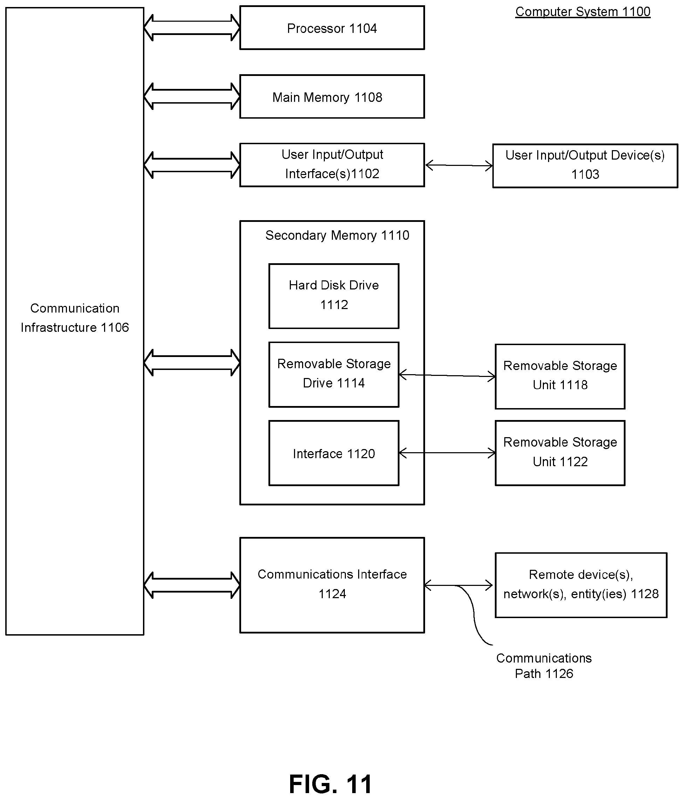

FIG. 11 is a block diagram of example components of a computer system 1100. One or more computer systems 1100 may be used, for example, to implement any of the embodiments discussed herein, as well as combinations and sub-combinations thereof, such as console 110. In some embodiments, one or more computer systems 1100 may be used to implement the methods, computing, and processing devices, as described herein. Computer system 1100 may include one or more processors (also called central processing units, or CPUs), such as a processor 1104. Processor 1104 may be connected to a communication infrastructure or bus 1106.

Computer system 1100 may also include user input/output interface(s) 1102, such as monitors, keyboards, pointing devices, etc., which may communicate with communication infrastructure 1106 through user input/output interface(s) 1103.

One or more of processors 1104 may be a graphics processing unit (GPU). In an embodiment, a GPU may be a processor that is a specialized electronic circuit designed to process mathematically intensive applications. The GPU may have a parallel structure that is efficient for parallel processing of large blocks of data, such as mathematically intensive data common to computer graphics applications, images, videos, etc.

Computer system 1100 may also include a main or primary memory 1108, such as random access memory (RAM). Main memory 1108 may include one or more levels of cache. Main memory 1108 may have stored therein control logic (i.e., computer software) and/or data. In some embodiments, main memory 1108 may include optical logic configured to perform processing and analysis of optical measurements obtained from tissue by a catheter and determine lesion predictions.

Computer system 1100 may also include one or more secondary storage devices or memory 1110. Secondary memory 1110 may include, for example, a hard disk drive 1112 and/or a removable storage drive 1114.

Removable storage drive 1114 may interact with a removable storage unit 1118. Removable storage unit 1118 may include a computer-usable or readable storage device having stored thereon computer software (control logic) and/or data. Removable storage unit 1118 may be a program cartridge and cartridge interface (such as that found in video game devices), a removable memory chip (such as an EPROM or PROM) and associated socket, a memory stick and USB port, a memory card and associated memory card slot, and/or any other removable storage unit and associated interface. Removable storage drive 1114 may read from and/or write to removable storage unit 1118.

Secondary memory 1110 may include other means, devices, components, instrumentalities, or other approaches for allowing computer programs and/or other instructions and/or data to be accessed by computer system 1100. Such means, devices, components, instrumentalities, or other approaches may include, for example, a removable storage unit 1122 and an interface 1120. Examples of the removable storage unit 1122 and the interface 1120 may include a program cartridge and cartridge interface (such as that found in video game devices), a removable memory chip (such as an EPROM or PROM) and associated socket, a memory stick and USB port, a memory card and associated memory card slot, and/or any other removable storage unit and associated interface.

Computer system 1100 may further include a communication or network interface 1124. Communication interface 1124 may enable computer system 1100 to communicate and interact with any combination of external devices, external networks, external entities, etc. (individually and collectively referenced by reference number 1128). For example, communication interface 1124 may allow computer system 1100 to communicate with external or remote devices 1128 over communications path 1126, which may be wired and/or wireless (or a combination thereof), and which may include any combination of LANs, WANs, the Internet, etc. Control logic and/or data may be transmitted to and from computer system 1100 via communication path 1126. In some embodiments, computer system 1100 may be coupled to a catheter via a connector and optical and electrical connections at communication interface 1124, including optical fibers and electrical wiring, pins, and/or components.

Computer system 1100 may also be any of a personal digital assistant (PDA), desktop workstation, laptop or notebook computer, netbook, tablet, smartphone, smartwatch or other wearables, appliance, part of the Internet-of- Things, and/or embedded system, to name a few non-limiting examples or any combination thereof.

Computer system 1100 may be a client or server, accessing or hosting any applications and/or data through any delivery paradigm, including but not limited to remote or distributed cloud computing solutions; local or on-premises software ("on-premise" cloud-based solutions); "as a service" models (e.g., content as a service (CaaS), digital content as a service (DCaaS), software as a service (SaaS), managed software as a service (MSaaS), platform as a service (PaaS), desktop as a service (DaaS), framework as a service (FaaS), backend as a service (BaaS), mobile backend as a service (MBaaS), infrastructure as a service (IaaS), etc.); and/or a hybrid model including any combination of the foregoing examples or other services or delivery paradigms.

Any applicable data structures, file formats, and schemas in computer system 1100 may be derived from standards including but not limited to JavaScript Object Notation (JSON), Extensible Markup Language (XML), Yet Another Markup Language (YAML), Extensible Hypertext Markup Language (XHTML), Wireless Markup Language (WML), MessagePack, XML User Interface Language (XUL), or any other functionally similar representations alone or in combination. Alternatively, proprietary data structures, formats, or schemas may be used, either exclusively or in combination with known or open standards.

In some embodiments, a tangible, non-transitory apparatus or article of manufacture comprising a tangible, non-transitory computer useable or readable medium having control logic (software) stored thereon may also be referred to herein as a computer program product or program storage device. This includes, but is not limited to, computer system 1100, main memory 1108, secondary memory 1110, and removable storage units 1118 and 1122, as well as tangible articles of manufacture embodying any combination of the foregoing. Such control logic, when executed by one or more data processing devices (such as computer system 1100), may cause such data processing devices to operate as described herein.

It is to be appreciated that the specification is intended to be used to interpret the claims. The Abstract section may set forth one or more but not all exemplary embodiments of the present disclosure as contemplated by the inventor(s), and thus, are not intended to limit the present disclosure and the appended claims in any way.

Embodiments of the present disclosure have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the disclosure that others can, by applying knowledge within the skill of the art, readily modify and/or adapt for various applications such specific embodiments, without undue experimentation, without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for description and not of limitation, such that the terminology or phraseology of the present specification is to be interpreted by the skilled artisan in light of the teachings and guidance.

The breadth and scope of the present disclosure should not be limited by any of the above-described exemplary embodiments but should be defined only in accordance with the following claims and their equivalents.

## Claims

1. A method, comprising:
generating a beam of coherent light;
coupling the beam of coherent light into an optical interferometer, wherein the optical interferometer is configured to:
deliver a first portion of the beam of coherent light to a tissue; and
provide an output signal based on an interference between a reflected light from the tissue and a second portion of the beam of coherent light;
determining a polarization of the beam of coherent light;
determining a birefringence of the tissue based on the output signal;
determining a configuration of a fiber alignment of the tissue based on the birefringence of the tissue and the polarization of the beam of coherent light; and
determining, according to the configuration of the fiber alignment of the tissue, an ablation parameter for ablating the tissue.

2. The method of claim 1, wherein determining the ablation parameter comprises determining an ablation parameter of a pulsed field for ablating the tissue.

3. The method of claim 2, wherein the ablation parameter is an energy, a duration, or a pulsed electric field vector of the pulsed field.

4. The method of any of the preceding claims, wherein determining the configuration of the fiber alignment of the tissue comprises determining the configuration of the fiber alignment at different depths within the tissue.

5. The method of any of the preceding claims, wherein determining the configuration of the fiber alignment of the tissue comprises generating a three-dimensional (3D) configuration of the fiber alignment of the tissue.

6. The method of any of the preceding claims, wherein determining the birefringence of the tissue comprises calculating the birefringence at different depths in the tissue.

7. The method of any of the preceding claims, wherein the optical interferometer comprises a sample arm guiding the first portion of the beam of coherent light and a reference arm guiding the second portion of the beam of coherent light.

8. The method of claim 7, wherein the sample arm comprises an optical multiplexer and a plurality of optical paths.

9. The method of claim 8, further comprising selecting, using the optical multiplexer, an optical path of the plurality of optical paths to deliver the first portion of the beam of coherent light to the tissue.

10. A method, comprising:
generating a beam of coherent light;
coupling the beam of coherent light into an optical interferometer, wherein the optical interferometer is configured to:
deliver a first portion of the beam of coherent light to a tissue; and
provide an output signal based on an interference between a reflected light from the tissue and a second portion of the beam of coherent light;
adjusting a polarization of the beam of coherent light;
determining a change in the output signal when adjusting the polarization of the beam of coherent light;
determining a configuration of a fiber alignment of the tissue according to the change of the output signal and the polarization of the beam of coherent light; and
determining, according to the configuration of the fiber alignment of the tissue, an ablation parameter for ablating the tissue.

11. The method of claim 10, wherein determining the configuration of the fiber alignment of the tissue comprises calculating a birefringence of the tissue as a function of the polarization of the beam of coherent light.

12. The method of claim 11, wherein determining the configuration of the fiber alignment of the tissue comprises calculating the birefringence of the tissue at different depths of the tissue.

13. The method of any of claims 10-12, wherein determining the configuration of the fiber alignment of the tissue comprises generating a three-dimensional (3D) configuration of the fiber alignment of the tissue.

14. The method of any of claims 10-13, wherein determining the ablation parameter comprises determining an ablation parameter of a pulsed field for ablating the tissue.

15. The method of claim 14, wherein the ablation parameter is an energy, a duration, or a pulsed electric field vector of the pulsed field.

16. The method of any of claims 10-15, wherein the optical interferometer comprises a sample arm guiding the first portion of the beam of coherent light and a reference arm guiding the second portion of the beam of coherent light.

17. The method of claim 16, wherein the sample arm comprises an optical multiplexer and a plurality of optical paths.

18. The method of claim 17, further comprising selecting, using the optical multiplexer, an optical path of the plurality of optical paths to deliver the first portion of the beam of coherent light to the tissue.

19. The method of any of claims 18, further comprising:
measuring a first output signal while selecting a first optical path; and
measuring a second output signal while selecting a second optical path.

20. The method of any of claims 19, wherein determining a configuration of a fiber alignment of the tissue comprises comparing the first and second output signals.

21. The method of any of claims 10-20, further comprising determining a polarization of the first portion of the beam of coherent light.

22. A method, comprising:
generating a beam of coherent light;
coupling the beam of coherent light into an optical interferometer, wherein the optical interferometer is configured to:
deliver a first portion of the beam of coherent light, via an optical port, to a tissue; and
provide an output signal based on an interference between a reflected light from the tissue and a second portion of the beam of coherent light;
determining a polarization of the beam of coherent light;
adjusting a position of the optical port concerning the tissue;
determining a change in the output signal when adjusting the position of the optical port;
determining a configuration of a fiber alignment of the tissue according to the change of the output signal and the polarization of the beam of coherent light; and
determining, according to the configuration of the fiber alignment of the tissue, an ablation parameter for ablating the tissue.

23. The method of claim 22, wherein determining the configuration of the fiber alignment of the tissue comprises calculating the birefringence of the tissue at different depths of the tissue.

24. The method of any of claims 22 or 23, wherein determining the configuration of the fiber alignment of the tissue comprises generating a three-dimensional (3D) configuration of the fiber alignment of the tissue.

25. The method of any of claims 22-24, wherein determining the ablation parameter comprises determining an ablation parameter of a pulsed field for ablating the tissue.

26. The method of claim 25, wherein the ablation parameter is a power, a duration, or a pulsed electric field vector of the pulsed field.

27. The method of any of claims 22-26, wherein the optical interferometer comprises a sample arm guiding the first portion of the beam of coherent light and a reference arm guiding the second portion of the beam of coherent light.

28. The method of any of claims 22-27, further comprising determining a polarization of the first portion of the beam of coherent light.
